# FASCICULE DE BREVET EUROPEEN

(11) **EP 0 581 938 B1**
(45) Date de publication et mention de la délivrance du brevet: **02.05.1997**
(21) Numéro de dépôt: 93904070.5
(22) Date de dépôt: 11.01.1993
(51) Int. Cl.: C12Q 1/68

(54) **PROCEDE DE DETECTION DE MUTATIONS PAR ELECTROPHORESE EN GRADIENT DE DENATURATION D'ADN DOUBLE BRIN STABILISE PAR PHOTOPONTAGE**
Verfahren zum Nachweis von Mutationen durch Denstrisierungsgradient-Elektrophorese von durch Photoüberbrückung stabilisierter doppelsträngiger DNS
MUTATION DETECTING METHOD USING PHOTOBRIDGING-STABILISED DOUBLE-STRANDED DNA DENATURATION GRADIENT ELECTROPHORESIS

(30) Priorité: 24.01.1992 FR 9200737
(43) Date de publication de la demande: 09.02.1994
(73) Titulaire: DUPRET, Daniel, F-67590 Schweighouse-sur-Moder (FR)
(72) Inventeur: DUPRET, Daniel, F-67500 Haguenau (FR); GOOSSENS, Michel, F-75014 Paris (FR); CHASSIGNOL, Marcel, F-45400 Semoy (FR); NGUYEN, Thank, Thuong, F-45510 Vienne-en-Val (FR)
(74) Mandataire: Kopacz, William James
(86) Numéro de dépôt international: FR9300020
(87) Numéro de publication internationale: WO9315223

(56) Documents cités:
- EP-A- 0 117 777
- WO-A-89/02930
- WO-A-90/01563
- PROCEEDINGS OF THE NATIONAL ACADEMY OF SCIENCES OF USA, vol. 86, Janvier 1989, WASHINGTON, US; V.C. SHEFFIELD ET AL, pages 232-236

## Description

La présente invention concerne un nouveau procédé de détection de mutations par électrophorèse sur gel en gradient de dénaturation, d'ADN double brin stabilisé par photopontage au moyen d'un ou plusieurs agents intercalants photoactivés.

L'invention concerne plus particulièrement l'application d'oligonucléotides liés à au moins un agent intercalant photoactivable pouvant former sous irradiation des produits de pontages avec les séquences nucléiques complémentaires, en tant qu'amorces pour stabiliser les hybrides formés lors d'un processus d'amplification génétique, en vue de l'analyse desdits hybrides par électrophorèse de retardement sur gel en gradient de dénaturation.

La détection de mutations selon des méthodes par retardement sur gel en gradient de dénaturation repose sur le fait que la mobilité électrophorétique (vitesse de migration) d'un fragment d'ADN est différente selon que le fragment est complètement dénaturé (simple brin), partiellement dénaturé (boucles ou épingles), ou parfaitement apparié (double brin). Un ADN double brin peut être dénaturé par élévation de température; des agents dénaturants tels que l'urée ou la formamide favorisent cette dénaturation et permettent d'abaisser la température de fusion de l'hybride.

Lorsqu'un fragment d'ADN double brin est soumis à des conditions de dénaturation progressive, on observe une dénaturation par palier de l'hybride. La dénaturation progressive aura pour effet l'ouverture de différents domaines de la chaîne d'ADN double brin, en commençant par les domaines les moins stables, jusqu'à la dissociation complète de l'hybride. Il a été montré que les zones riches en paires G/C sont plus stables que les zones riches en paires A/T.

Des boucles ou des épingles apparaîtront donc dans les domaines ou l'instabilité sera atteinte aux conditions de dénaturation données.

La migration électrophorétique dans un gel d'acrylamide où l'on a créé un gradient de dénaturation, par exemple une variation de concentration d'agent dénaturant ou une variation de température, permet de discriminer un hybride parfait d'un hybride imparfait présentant une boucle de fusion ou une épingle, le premier étant dénaturé plus tard et migrant plus loin sur le gel, car la dissociation d'un domaine arrête ou ralenti considérablement la mobilité électrophorétique du fragment d'ADN.

Le gradient de dénaturation concomitant à l'électrophorèse peut être obtenu par au moins trois méthodes :
- un gradient de température le long du gel, méthode dénommée TGGE pour "Température Gradient Gel Electrophoresis";
- un gradient de dénaturant chimique le long du gel, méthode dénommée DGGE pour "Denaturant Gradient Gel Electrophoresis" (voir à ce sujet la référence 6);
- un gradient de température dans le temps, méthode dénommée TSGE pour "Temperature Sweep Gel Electrophoresis" (Kenji Yoshino et al., Nucleic Acids Research, Vol. 19, No. 11, 3153, 1991).

En réalisant une électrophorèse sur des gels d'acrylamide, dans ces conditions dénaturantes croissantes, il est possible de séparer par le ralentissement de la mobilité électrophorétique, les fragments présentant une dénaturation d'au moins un aine de ceux ne présentant pas de dénaturation, ou de ceux complètement dénaturés, et ainsi d'identifier des mutations ponctuelles jusqu'à une paire de bases (S. G. Fisher, L. S. Lerman, Proc. Natl. Acad. Sci. USA, Vol. 80, pp. 1579 - 1583, March 1983, Biochemistry); (O. Attree et al. Genomics 4, 266-272, 1989); (R. M. Myers et al. Nature Vol. 313, February 1985, pp. 495-497).

Le principe de ces techniques, bien que connues depuis longtemps, n'a pu être facilement mis en oeuvre que récemment grâce à l'apparition du procédé d'amplification génétique, qui permet d'obtenir aisément des fragments d'ADN pouvant être facilement analysés par ces techniques. L'amplification génétique consiste à effectuer un cycle constitué d'une phase d'hybridation, d'une phase d'extension et d'une phase de dénaturation, et à répéter ce cycle hybridation-extension-dénaturation, connu sous le nom de cycle PCR pour "Polymerase Chain Reaction", un nombre de fois suffisant pour augmenter la quantité du fragment d'ADN de départ dans une proportion exponentielle par rapport au nombre de cycles mis en oeuvre.

La phase d'hybridation consiste à utiliser deux oligonucléotides amorces, complémentaires chacun d'une séquence d'environ 20 bases située en (3') de part et d'autre du fragment d'ADN à amplifier; le mélange des deux amorces avec de l'ADN génomique, dans des conditions d'hybridation appropriées, permet de positionner chaque amorce en face de leur séquence complémentaire sur le fragment d'ADN.

La phase d'extension consiste à faire agir une ADN polymèrase pour laquelle les extrémités (3') des oligonucléotides servent d'amorces et chaque brin d'ADN du fragment à amplifier sert de matrice. Chaque oligonucléotide amorce est allongé dans le sens (5')-(3') jusqu'à obtenir un doublement de la séquence du fragment à amplifier.

La phase de dénaturation consiste à séparer par l'action de la chaleur le double brin de l'hybride issu de la phase d'extension avant la mise en oeuvre de la phase d'hybridation suivante.

L'amplification génétique par PCR permet, grâce aux techniques de retardement sur gel dénaturant, d'étudier de manière très précise dans les populations la présence de mutations, afin de correler celles-ci avec la présence de maladies génétiques (R. K. Saiki et al., Nature, Vol. 324, 13 November 1986, PP163-166), (C. Wong et al., Nature, Vol. 384, 26 November 1987, pp 384-386).

En effet, supposons une région du génome susceptible de porter une mutation. Connaissant sa séquence, des modèles de calcul permettent d'identifier dans le fragment d'ADN le ou les domaines de fusion successifs, et ainsi de prévoir leur température de fusion (voir à ce sujet la référence 10); il est aussi possible de prévoir l'influence de mutations même ponctuelles sur la température de fusion des domaines en question (voir à ce sujet la référence 10).

Grâce à l'amplification génétique par PCR, il est possible, en choisissant correctement une paire d'oligonucléotides amorces, d'amplifier un fragment d'ADN contenant au moins deux domaines de fusion et d'obtenir une quantité importante (quelques centaines de nanogrammes) de ce fragment purifié. L'analyse électrophorétique de ce fragment dans un gradient de dénaturation chimique ou thermique permet de mesurer la température de fusion du domaine; celle-ci variant avec la séquence, il est possible d'identifier la présence éventuelle de mutations, sur la base d'une migration différente du fragment variant par rapport au fragment témoin.

Une étape importante de ce protocole réside dans le choix des oligonucléotides amorces spécifiques qui encadrent le domaine de fusion étudié.

En effet, une variation nucléotidique ne peut être détectée que si elle est située dans le premier domaine de fusion, c'est à dire le segment qui se dénature le premier lorsque le fragment rencontre, dans le gel, l'endroit où la concentration de dénaturant (ou la température) correspond à sa température de fusion (Tm). Une mutation n'est détectable par ce procédé que s'il existe un deuxième domaine, plus stable que le premier et que si elle est localisée dans le premier domaine de fusion. Afin de positionner la séquence d'ADN explorée dans le premier domaine de fusion de façon systématique, on propose dans l'art antérieur d'analyser la séquence du duplex d'ADN à l'aide d'algorithmes appropriés afin de déterminer la position des amorces d'amplification, et de stabiliser l'une des extrémités du fragment analysé en introduisant, à l'aide d'oligonucléotides amorces modifiés, une queue GC d'au moins 40 bases, aussi appelée "GC clamp". L'introduction de ce GC clamp crée un domaine résistant à la dénaturation, ce qui rend le (ou les) domaine(s) moins stable(s) analysable(s) pour les mutations qu'il contient.

On propose dans l'art antérieur de stabiliser l'une des extrémités du fragment amplifié à l'aide d'oligonucléotides amorces modifiés en introduisant en position (5') d'une queue GC (Kenji Yoshino et al., Nucleic Acids Research, Vol. 19, No. 11, 3153, 1991) (voir aussi à ce sujet la référence 15).

Ce modèle est notamment couramment utilisé par des laboratoires spécialisés pour le diagnostic de mutations dans les gènes de la bêta globine, des facteurs IX et VIII de la coagulation, et de la mucoviscidose (voir à ce sujet les références 2, 4, 7, 8, 9, 16, 17).

Or, la synthèse de GC clamp est difficile à réaliser car la désoxyguanine (représentée par la lettre G), qui est une purine, est également le nucléoside le plus fragile dans les opérations de synthèses des oligonucléotides. En effet, au cours de chaque cycle de couplage par la méthode des phosphoramidites, lors de la synthèse des oligonucléotides, une opération de détrytilation en milieu acide par le TCA est effectuée; et, en fin de synthèse, un traitement basique par l'ammoniac à chaud est également réalisée pour la déprotection des bases. On observe alors qu'une grande partie des oligonucléotides, surtout lorsqu'ils comprennent de nombreux G, sont tronqués précisément au niveau des G du fait d'une dépurination.

Les GC clamp utilisés dans ces techniques sont souvent très longs et peuvent dans certains cas atteindre 60 à 80 bases. Leur synthèse est donc délicate, entraînant un surcoût considérable dans la préparation d'oligonucléotides amorces.

En outre, les GC clamp ne sont pas toujours assez stables pour éviter l'ouverture de l'extrémité avant la fusion du domaine, et en conséquence ne permettent pas l'étude de certains domaines de fusion.

La présente invention vise à fournir un nouveau moyen de stabilisation d'au moins une extrémité du fragment d'ADN à étudier, ne présentant pas les inconvénients des GC clamp. Ce but est atteint selon l'invention en remplaçant le GC clamp par l'introduction d'au moins une liaison covalente entre les deux brins d'ADN au niveau de l'extrémité à stabiliser.

La Demande de Brevet Français publiée sous le N° 2 540 122 décrit à titre de nouveaux composés chimiques, une séquence d'oligonucléotides liée à un agent intercalant. Ces composés chimiques se lient de façon sélective à toute séquence complémentaire d'oligonucléotides. La présence d'un agent intercalant qui possède une forte affinité pour les plateaux de bases permet de stabiliser l'hybride formé qui cependant peut être dissocié. En outre, la présence des agents intercalants dotés de propriétés distinctes des nucléotides permet leur détection.

Les agents intercalants proposés dans la Demande de Brevet mentionnée ci-dessus sont des composés connus dans les techniques touchant aux acides nucléiques, il s'agit en général de composés polycycliques ayant une configuration plane tels que l'acridine, la furocoumarine ou l'ellipticine et leurs dérivés.

Parmi ceux-ci, le psoralène (ou furocoumarine) et ses dérivés, désignés ci-après par agent intercalant photoactivable, outre leur capacité à s'intercaler entre les plateaux de bases des deux brins d'ADN, présentent la propriété de former des liaisons covalentes entre la double liaison 3-4 du cycle pyrone ou 4'-5' du cycle furane, avec la double liaison 5,6 des bases pyrimidiques, en particulier la thymine, sous irradiation à environ 360 nm.

Ainsi l'agent intercalant photoactivable couplé à un oligonucléotide par l'intermédiaire d'un bras carboné adéquat, comme décrit dans la Demande de Brevet Français n° 2 540 122, s'intercalera entre les plateaux de bases formés par l'oligonucléotide et toute séquence d'acide nucléique complémentaire; l'irradiation à 360 nm des hybrides obtenus permettra de créer des liaisons covalentes entre l'agent intercalant ainsi activé et la séquence d'acide nucléique complémentaire de manière à créer un hybride ponté stable.

On connait également la Demande de Brevet Internationale publiée sous le n° WO 90/12020 qui propose de coupler la furocoumarine à un oligonucléotide par l'intermédiaire d'un sucre ribose ou déoxyribose.

Les Demandes de Brevet Européenne n° 316 016, Internationale n° WO 89/06702 et Allemande n° 3928900 décrivent l'utilisation de conjugués de psoralène et d'oligonucléotides pour bloquer l'expression génétique.

La demande de Brevet Français n° 2 568 254, décrit l'application de composés oligonucléotides liés à un agent intercalant pour le blocage sélectif d'une séquence d'acide nucléique, et plus particulièrement l'application de ces composés au blocage sélectif *in vivo* de l'expression d'un gène ou d'une séquence impliquée dans l'initiation, la propagation ou la terminaison de la réplication d'un acide nucléique, de la transcription d'un ou plusieurs gènes et/ou de leur traduction.

La Demanderesse a maintenant mis en évidence que des oligonucléotides couplés à au moins un agent intercalant photoactivable, tel que le psoralène ou ses dérivés, pouvaient être utilisés comme amorces dans des processus d'amplification génétique *in vitro* par PCR, et que les hybrides ainsi obtenus étaient, après photopontage, parfaitement stabilisés pour être étudiés en électrophorèse de retardement sur gel en gradient de dénaturation.

L'invention concerne en conséquence un procédé de détection de mutations par électrophorèse d'ADN double brin en gradient de dénaturation, caractérisé en ce que l'hybride analysé est stabilisé par photopontage au moyen d'un ou plusieurs agents intercalants photoactivés.

Selon le procédé de l'invention, l'hybride analysé est obtenu par un processus d'amplification génétique d'un fragment d'ADN double brin, dont les phases d'hybridation sont effectuées avec deux oligonucléotides amorces sur l'un desquels au moins, se trouve fixé à l'extrémité (5'), un ou plusieurs agents intercalants photoactivables.

Après le processus d'amplification génétique par PCR, l'irradiation à environ 360 nm des hybrides obtenus permet de créer des liaisons covalentes entre le ou les agents intercalants ainsi activés et les bases pyrimidiques, en particulier les thymines, se trouvant dans le voisinage immédiat sur le brin complémentaire. Ces liaisons covalentes assurent un pontage stable entre le brin d'ADN porteur d'au moins un agent intercalant photoactivable et le brin d'ADN matrice sur lequel le pontage a été réalisé.

L'ensemble constitué d'un oligonucléotide amorce auquel est fixé au moins un agent intercalant photoactivable répond à la formule suivante : 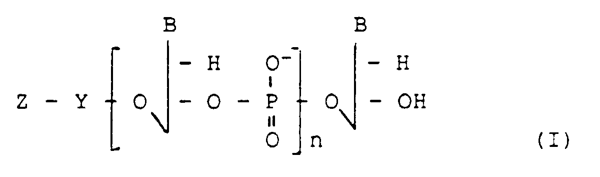 dans laquelle :
Les radicaux B peuvent être identiques ou différents et représentent chacun une base d'un acide nucléique.
Y représente un radical alcoylene droit ou ramifié - alk - ou un radical : 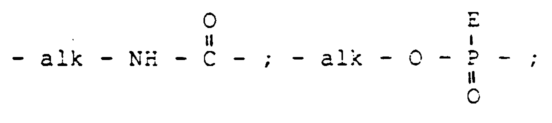 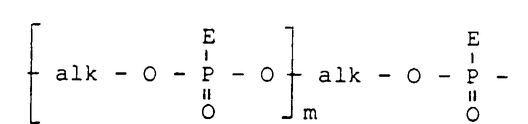
Les radicaux E peuvent être identiques ou différents et représentent chacun un oxoanion O⁻, un thioanion S⁻, un groupe alcoxy, ou un groupe - O - alk - Z; et m est un nombre entier compris entre 1 et 5.
Z est un radical correspondant à un agent intercalant photoactivable pouvant former des photopontages avec les bases pyrimidiques.
n est un nombre entier compris entre 2 et 150.

Il convient de remarquer que la formule I représente un enchaînement de nucléotides, dont la séquence est complémentaire d'une séquence de 15 à 30 bases située à l'une des extrémité (3') du fragment d'ADN à amplifier; n indiquant simplement le nombre de nucléotides compris dans la molécule; n est un nombre compris entre 2 et 150 et de préférence entre 10 et 30.

Les agents intercalants Z photoactivables pouvant former des photopontages avec les bases pyrimidiques, sont des composés connus dans les techniques touchant aux acides nucléiques, il s'agit de composés capables de "s'intercaler" dans la structure des ADN ou des ARN en double hélice, sur des hybrides ADN-ARN, et de former des produits de pontage avec les bases pyrimidiques sous irradiation.

Ces agents intercalants sont, en général, ces composés polycycliques ayant une configuration plane en possédant des doubles liaisons photoactivables, ou un groupe azido-N₃. Parmi ces composés on peut citer par exemple les dérivés du 8-méthoxypsoralène, du 5-méthoxypsoralène, du 4'hydroxyméthyl-4,5',8-triméthylpsoralène, du 3-carbethoxypsoralène, de l'angelicine, du pyrido(3,4-c)psoralène, du pyrido (3,4-c)8-méthylpsoralène.

Parmi les significations de Z, deux seront utilisées plus particulièrement :
- le groupe oxy-8-psoralène,
- le groupe oxy-5-psoralène.

Ces composés peuvent être préparés par des procédés qui sont déjà connus (F. Eckstein. In Oligonucleotides an analogues : a practical; Oxford University Press (1991), pp. 283-308) en particulier par le procédé de synthèse dit "au phosphoramidite".

Dans la formule (I), on utilise la représentation condensée des nucléotides suivante : 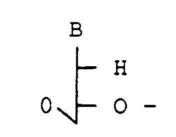 qui correspond à la formule développée : 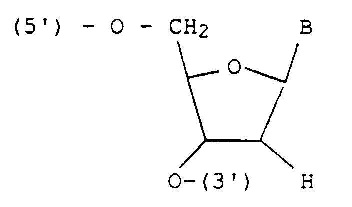 sur laquelle ont été mentionnées les extrémités (3') et (5').

Selon un premier mode de réalisation, on préfère dans la formule (I) que le radical B situé à l'extrémité (5') de l'oligonucléotide amorce soit une adénine (A). Selon en second mode de réalisation, on préfère dans la formule (I) que au moins deux des radicaux B successifs situés à l'extrémité (5') de l'oligonucléotide amorce soient des adénines.

En effet, l'irradiation de l'hybride obtenu après amplification, permettra de créer des liaisons covalentes entre l'agent intercalant ainsi activé et les thymines (T) se trouvant dans le voisinage immédiat sur le brin complémentaire.

De manière avantageuse, le radical Z est choisi parmi le groupe oxy-8-psoralène et oxy-5-psoralène.

Selon une forme de mise en oeuvre préférée du procédé de l'invention, l'un seulement des oligonucléotides amorces est conjugué à un agent intercalant photoactivable.

Le procédé de détection de mutations selon l'invention consiste tout d'abord à effectuer une amplification génétique, dont les phases d'hybridation sont effectuées avec deux oligonucléotides amorces sur l'un desquels au moins se trouve fixé un ou plusieurs groupes intercalants photoactivables, du fragment d'ADN à analyser, puis en ce que l'on soumet les hybrides obtenus à une irradiation, et en ce que l'on détecte d'éventuelles mutations en comparant le résultat de l'électrophorèse de retardement sur gel en gradient de dénaturation desdits hybrides préalablement irradiés avec l'électrophorèse dans les mêmes conditions d'un échantillon d'ADN témoin dépourvu de mutation ou comportant une mutation connue.

L'électrophorèse de retardement sur gel en gradient de dénaturation est effectuée de préférence selon un gradient de température ou un gradient de dénaturant chimique ou encore un gradient de température dans le temps.

L'invention a aussi pour objet l'application d'un oligonucléotide à l'extrémité (5') duquel se trouve fixé au moins un agent intercalant photoactivable, pour l'amplification génétique d'un fragment d'ADN, dans laquelle l'extrémité (3') de l'oligonucléotide sert d'amorce à une ADN polymèrase.

L'invention a encore pour objet l'application d'un hybride stabilisé par photopontage au moyen d'au moins un agent intercalant photoactivé, pour la détection de mutations par électrophorèse de retardement sur gel en gradient de dénaturation, dans laquelle l'hybride a été préparé par un processus d'amplification génétique d'un fragment d'ADN double brin dont les phases d'hybridation sont effectuées avec deux oligonucléotides amorces sur l'un desquels au moins se trouve fixé au moins un agent intercalant photoactivable.

Outre les caractéristiques qui précèdent, l'invention comporte d'autres caractéristiques qui apparaîtront au cours de la description qui suit et qui se référent à des exemples de réalisation et de mise en oeuvre de la présente invention, étant entendu que ces exemples ne sauraient constituer une quelconque limitation à la portée des revendications.

### I - PREPARATION DES AMORCES

L'exemple suivant concerne l'amplification génétique par PCR d'une partie de l'exon 10 du gène de la mucoviscidose (CFTR). On utilise à titre d'amorces, les deux oligonucléotides de 20 bases chacun, de séquences suivantes :

Amorce 1 : d-(5') G A A C T G G A G C C T T
C A G A G G G (3')
Amorce 2 : d-(3') G C A G T A G T T T C G T
A C G G T T G (5')

Les deux amorces complémentaires d'une courte séquence située aux extrémités (3') des brins du fragment d'ADN correspondant à la partie étudiée de l'exon 10 sont mis à hybrider avec ledit fragment. Les extrémités 3'OH des oligonucléotides servent d'amorces à une ADN polymèrase; chaque brin du fragment sert de matrice à l'enzyme qui synthétise les brins complémentaires.

La séquence de la partie étudiée de l'exon 10 est indiquée ci-dessous encadrée par les deux oligonucléotides amorces.

d-(5') GAACTGGAGCCTTCAGAGGGTAAAATTAAGCAC
d-(3') CTTGACCTCGGAAGTCTCCCATTTTAATTCGTG

AGTGGAAGAATTTCATTCTGTTCTCAGTTTTCCTGGA
TCACCTTCTTAAAGTAAGACAAGAGTCAAAAGGACCT

TTATGCCTGGCACCATTAAAGAAAATATCATCTTTGGT
AATACGGACCGTGGTAATTTCTTTTATAGTAGAAACCA

GTTTCCTATGATGAATATAGATACAGAAGCGTCATCAA
CAAAGGATACTACTTATATCTATGTCTTCGCAGTAGTT

AGCATGCCAAC (3')
TCGTACGGTTG (5')

Pour stabiliser les hybrides formés après n cycles hybridation-extension-dénaturation du processus PCR, on utilise dans l'art antérieur un clamp GC de 40 bases greffé à l'extrémité (5') de l'amorce 1.

Cette queue GC présente par exemple la séquence suivante :

d (5')G C C C G C C G T C C C G G C C C G A
C C C C C G C  G C G T C C G G C G C C C G

L'invention propose de remplacer cette queue GC par un groupe oxy-5-psoralène; lequel est couplé à l'extrémité 5' de l'amorce 1 par l'intermédiaire d'un groupe hexyle-phosphodiester de formule :

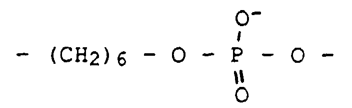

Afin de favoriser, lors de l'irradiation, la formation de liaisons covalentes entre le psoralène et les deux nucléotides T T complémentaires des nucléotides A A de l'amorce 1, on a supprimé le premier nucléotide G en (5') de ladite amorce 1.

### II- AMPLIFICATION GENETIQUE PAR PCR

L'étude suivante vise à montrer d'une part, que la présence du psoralène sur l'amorce 1 ne perturbe pas le processus d'amplification, et qu'un fragment unique est correctement synthétisé et d'autre part, l'efficacité du photopontage.

### 1) Amplification

Mélange réactionnel :
- ADN génomique 250 ng
- Amorce 1 400 pmoles
- Amorce 2 400 pmoles
- dATP 0,4 mM
- dGTP 0,4 mM
- dCTP 0,4 mM
- dTTP 0,4 mM
- Tris HCl pH 9 50 mM
- KCl 50 mM
- (NH₄)₂SO₄ 16 mM
- MgCl₂ 7 m
- BSA 0,2 mg/ml
- Taq DNA polymèrase 2,5 U
- H₂O QSP 400 µl

Le mélange réactionnel est recouvert d'huile de paraffine, puis placé dans un incubateur programmable et soumis au cycles suivants :

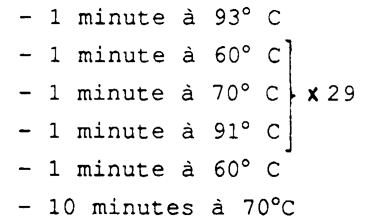

Puis l'huile de paraffine est enlevée.

### 2) Irradiation

100 µl de produit d'amplification est déposé sur une plaque de verre. L'irradiation est réalisée à l'aide d'une lampe UV de type Vilber Lourmat VL-4LC (365 nm) placée à 5 mm au dessus de l'échantillon, pendant une durée de 12 minutes.

### 3) Analyse des produits d'amplification et d'irradiation

### a) Analyse sur gel de polyacrylamide non dénaturant

10 µl de produit d'amplification non irradié en 10 µl du produit d'amplification irradié sont déposés avec un échantillon de marqueur de poids moléculaire (pBR 322/Hae III) sur un gel de polyacrylamide à 8 %.

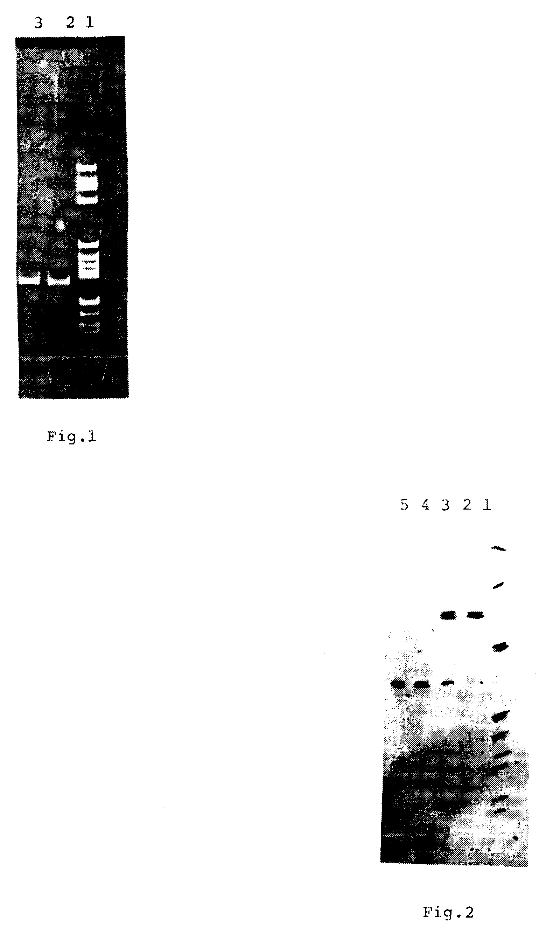

La figure 1, représente le résultat de l'électrophorèse, après traitement du gel au bromure d'éthidium :
- La piste 1 correspond au marqueur de poids moléculaire (pBR 322/Hae III).
- La piste 2 correspond au produit d'amplification non irradié.
- La piste 3 correspond au produit d'amplification irradié.

On observe au niveau des pistes 2 et 3 une bande unique d'environ 150 paires de bases. Ce résultat indique que l'amplification n'est pas perturbée par la présence du psoralène et qu'en outre aucune dégradation du fragment irradié n'est survenue.

### b) Analyse sur gel de polyacrylamide en conditions dénaturantes

L'analyse est effectuée sur un gel de polyacrylamide à 8 %, urée 8 M, électrophorèse thermostatée à 50 °C.

Le résultat de l'électrophorèse est représenté à la figure 2 :
- La piste 1 correspond au dépôt du marqueur de poids moléculaire (pBR 322/Hae III).
- La piste 2 correspond au dépôt de 10 µl d'échantillon irradié et dénaturé à 100° C pendant 5 minutes avant dépôt.
- La piste 3 correspond au dépôt de 10 µl de l'échantillon irradié non dénaturé.
- La piste 4 correspond au dépôt de 10 µl d'échantillon non irradié et dénaturé à 100° C pendant 5 minutes avant dépôt.
- La piste 5 correspond au dépôt de 10 µl d'échantillon non irradié et non dénaturé.

On constate que le produit d'amplification soumis à l'irradiation n'est pas dégradé, et que 80 à 90 % du fragment d'ADN présente un poids moléculaire du double de celui du fragment non irradié; ce qui signifie que le pontage à l'extrémité (5') du fragment irradié permet de révéler après dénaturation complète un ADN simple brin du double de la taille du fragment d'ADN entièrement apparié.

### III - EXEMPLES DE DETECTION DE MUTATIONS SELON LE PROCEDE DE L'INVENTION

### 1) Analyse d'une délétion de 3 paires de bases

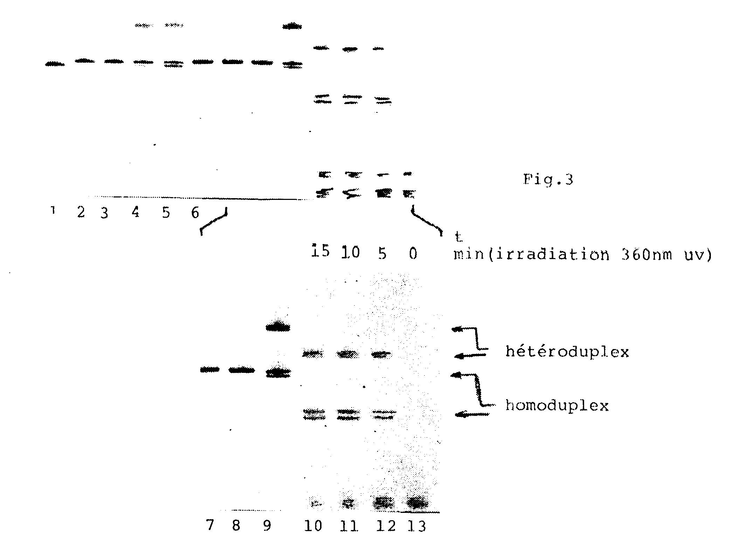

La figure 3 représente les résultats d'une analyse comparative en DGGE (gradient de dénaturant de 10% à 60%) d'échantillons d'ADN normal (Nl) et variant (ΔF 508) pour l'exon 10 du gène CFTR (mucoviscidose), en utilisant pour stabiliser l'hybride soit une queue GC, soit le psoralène; dans ce dernier cas, le psoralène est lié à l'extrémité 5' de l'acide nucléique par l'intermédiaire d'un bras constitué de 6 groupements méthylènes (CH₂)₆, cette structure est notée : Pso-m6, _{5'→3'.}

Le gradient de dénaturant de 10% à 60% est réalisée à partir d'une solution mère à 80% de dénaturant ayant la composition suivante 32% formamide, 5,6 M urée dans le TAE 1X (TAE 1X = Tris 40 mM, acétate de sodium 20 mM, EDTA 1 mM, pH 7,4). La concentration en acrylamide est de 6,5% (acrylamide/bisacrylamide : 37,5/1) . Le temps ce migration est de 3 heures, et le voltage appliqué est de 160V.
- La piste 1 correspond à un dépôt d'un échantillon contenant l'ADN variant ΔF 508; stabilisation des hybrides par GC clamp.
- Les pistes 2, 3, 6, 7 et 8 correspondent à des dépôts d'échantillons contenant l'ADN normal N1; stabilisation des hybrides par un GC clamp.
- Les pistes 4, 5 et 9 correspondent à des dépôts d'échantillons contenant en proportions égales les deux espèces Nl et ΔF 508; stabilisation des hybrides par GC clamp..
- Les pistes 10, 11 et 12 correspondent à des dépôts d'échantillons contenant chacun en proportions égales les deux espèces Nl et ΔF 508; stabilisation des hybrides par le psoralène après irradiation à 360 nm pendant respectivement 15, 10 et 5 minutes.
- La piste 13 correspond à un dépôt d'un échantillon contenant en proportions égales les deux espèces Nl et ΔF 508; stabilisation des hybrides par le psoralène sans irradiation de l'échantillon.

On observe avec la stabilisation par le psoralène des résultats comparables à ceux obtenus en utilisant les conditions de stabilisation de l'art antérieur avec le GC clamp. Il existe une bonne séparation des homoduplex et des hétéroduplex, ce qui permet une discrimination indiscutable des deux variants (allèle normal par rapport à l'allèle mutant ΔF 508). Seule varie la distance de migration par rapport à l'origine. On constate que 80 à 90 % du fragment d'ADN est modifié après action de l'irradiation à 360 nm.

### 2) Analyse d'une mutation ponctuelle

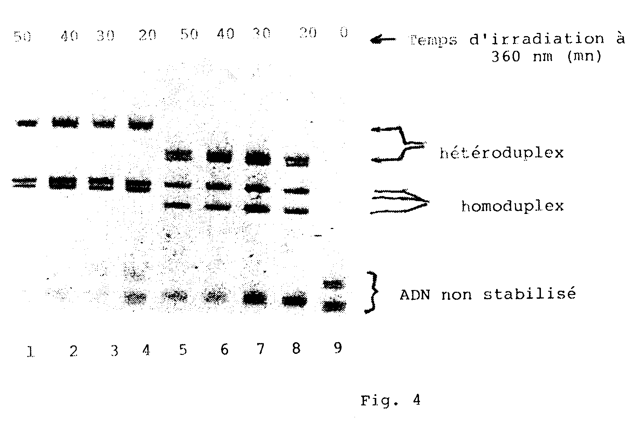

La figure 4 représente les résultats d'une analyse en DGGE (gradient de dénaturant 10 à 60 %, composition du gradient identique à celui utilisé pour l'analyse d'une délétion de 3 paires de bases décrite précédemment; 160 volts pendant 3 heures) d'échantillons d'ADN normal (Nl) et variants (ΔF 508 et I506V) pour l'exon 10 du gène CFTR (mucoviscidose), en utilisant pour stabiliser l'hybride le psoralène (Pso-m6, 5'->3').

Le variant I506V correspond à la substitution d'un nucléotide (nt 1648 A->G) dans l'exon 10 du CFTR.

Dans cette expérience, l'ADN a été amplifié par PCR à l'aide de deux amorces dont l'une contient un agent intercalant photoactivable, le psoralène, et exposé après amplification à des temps variables d'irradiation aux UV à 360 nm.
- Les pistes 1 à 4 correspondent à des dépôts d'échantillons contenant en proportions égales les deux espèces Nl et ΔF 508; stabilisation des hybrides par le psoralène après irradiation de l'échantillon à 360 nm respectivement pendant 50, 40, 30 et 20 minutes.
- Les pistes 5 à 9 correspondent à des dépôts d'échantillons contenant en proportions égales les deux espèces Nl et I506V; stabilisation des hybrides par le psoralène après irradiation à 360 nm respectivement pendant 50, 40, 30 et 20 minutes.

On observe qu'une substitution d'un nucléotide (nt 1648 A->G dans l'exon 10 du CFTR), présente à l'état hétérozygote dans un échantillon d'ADN humain est détectable facilement sur la présence d'homoduplex migrant différemment et d'hétéroduplex.

### 3) Analyse comparative dans un système d'électrophorèse perpendiculaire au gradient de dénaturant.

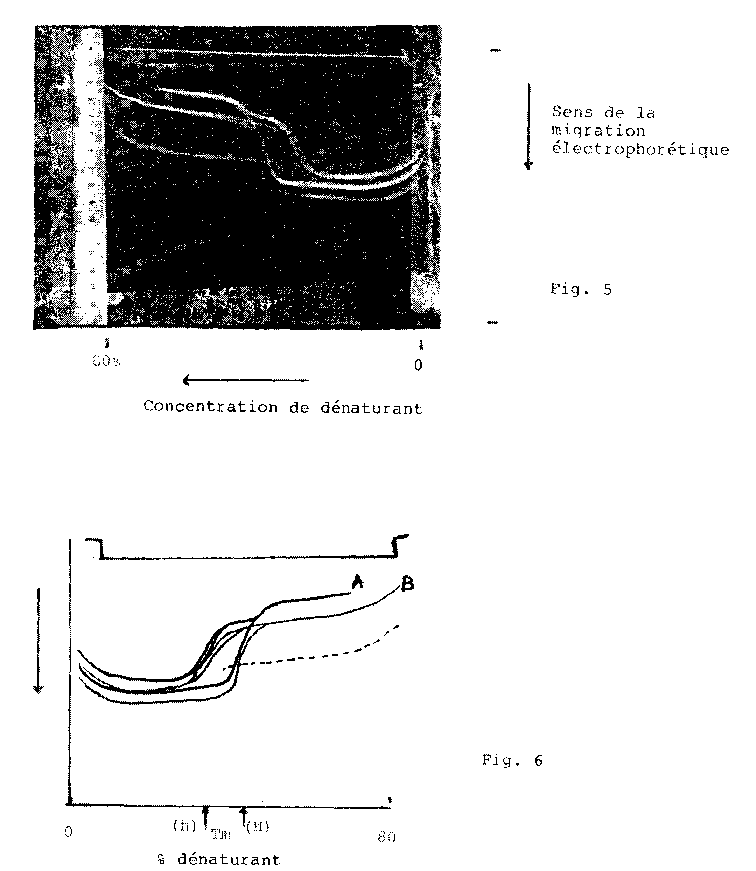

La figure 5 représente l'analyse, dans un système d'électrophorèse perpendiculaire au gradient de dénaturant de 0 à 80 %, de deux échantillons contenant chacun en proportions égales les deux espèces Nl et ΔF 508, et dont l'un est amplifié au préalable en présence d'une amorce portant une queue GC, l'autre étant amplifié avec une amorce couplée au psoralène.

Sur la figure 6, on a représenté en A la courbe de fusion de l'échantillon d'ADN amplifié en présence d'une queue GC, et en B la courbe de fusion de l'échantillon d'ADN amplifié avec une amorce couplée au psoralène; la courbe en pointillée correspond à l'ADN non stabilisé.

On constate que les courbes de fusion des deux échantillons, si elles ne sont pas superposables, présentent le même aspect. Notamment les pourcentages de dénaturant (Tm) pour lesquels 50 % des séquences sont dénaturés sont très voisins, qu'il s'agisse des homoduplex (H) ou des hétéroduplex (h).

### REFERENCES

1. Abrams ES, Murdaugh SE, Lerman LS; Genomics 1990; 7 : 463-475.
2. Attree O, Vidaud D, Vidaud M, Amselem S, Lavergne J, Goossens M; Genomics 1989; 4 : 266-272.
3. Burmeister M, Disibio G, Cox DR, Myers RM; Nucl. Acids Res. 1991; 19(7) : 1475-1480.
4. Devoto M, Ronchetto P, Fanen P, et al.; Am. J. Hum. Genet. 1991.
5. Fisher SG, Lerman LS; Cell 1979; 16 : 191.
6. Fisher SG, Lerman LS; Proc. Natl. Acad. Sci. 1983; 80 : 1579.
7. Goossens M, Fanen P, Attree O, Vidaud M; Ann. New York Acad. Sci. 1990; 612 : 74-80.
8. Kogan SC, Gitschier J; Blood 1988; 72 : 300 a.
9. Kogan SC, Gitschier J; Proc. Natl. Acad. Sci. USA 1990; 87(March) : 2092-2096.
10. Lerman LS, Silverstein K. In : Wu R, ed. Methods in Enzymology. New York; Academic Press, 1987 : 482-501. vol. 155.
11. Myers RM, Lumelsky N; Lerman L, Maniatis T; Nature 1985; 313 : 495-498.
12. Myers RM, Fisher SG, Lerman LS, Maniatis T; Nucl. Acids Res. 1985; 13 : 3131-3145.
13. Myers RM, Fisher SG, Maniatis T, Lerman LS; Nucl. Acids Res. 1985; 13 : 3111.
14. Myers RM, Sheffield VC, Cox DR. In : Ehrlich HA, ed. PCR technology : Principles and applications for DNA amplification. New York Stockton Press, 1989 : 71-88.
15. Scheffield VC, Cox DR, Lerman LS, Myers RM; Proc. Natl. Acad. Sci. USA, 1989; 86 : 232-236.
16.Traystman MD, Higuchi M, Kasoer C, Antonarakis SE, Kazazian HH; Genomics 1990; 6 : 293-301.
17.Vidaud M, Fanen P, Martin J, Ghanem N, Nicolas S, Goossens M; Hum. Genet. 1990; 85(4) : 446-449.
18.Weber CK, Shaffer DJ, Sidman CL; Nucl. Acids Res. 1991); 19(12) : 3331-3335.

## Revendications

1. Procédé de détection de mutations par électrophorèse d'ADN double brin en gradient de dénaturation, caractérisé en ce que l'une au moins des extrémités des fragments d'ADN double brin analysés est stabilisée par photopontage au moyen d'un ou plusieurs agents intercalants photoactivés, ladite stabilité par photopontage de l'une au moins des extrémités desdits ADN étant supérieure à celle d'au moins un domaine de dénaturation portant la ou les mutations analysées éventuellement présent dans l'ADN.

2. Procédé selon la revendication 1, caractérisé en ce que les fragments d'ADN double brin analysés sont obtenus par un processus d'amplification génétique d'un fragment d'ADN double brin, dont les phases d'hybridation sont effectuées avec deux oligonucléotides amorces sur l'un desquels au moins se trouve fixé un ou plusieurs agents intercalants photoactivables.

3. Procédé selon la revendication 2, caractérisé en ce que l'ensemble constitué d'un oligonucléotide amorce auquel est fixé un ou plusieurs agents intercalants photoactivables répond à la formule suivante : 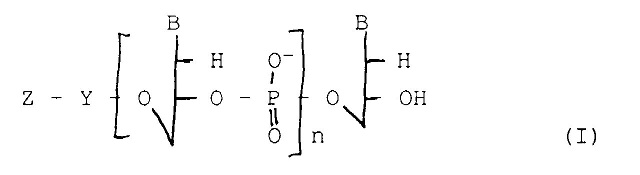 dans laquelle :
Les radicaux B peuvent être identiques ou différents et représentent chacun une base d'un acide nucléique.
Y représente un radical alcoylene droit ou ramifié - alk - ou un radical : 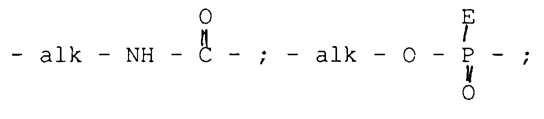 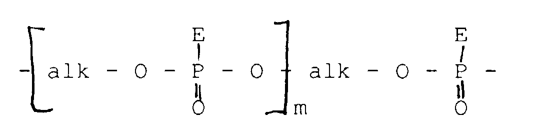
Les radicaux E peuvent être identiques ou différents et représentent chacun un oxoanion O⁻, un thioanion S-, un groupe alcoxy, ou un groupe - O - alk -Z; et m est un nombre entier compris entre 1 et 5.
Z est un radical correspondant à un agent intercalant photoactivable pouvant former des photopontages avec les bases pyrimidiques.
n est un nombre entier compris entre 2 et 150.

4. Procédé selon la revendications 3, caractérisé en ce que le radical Z est un composé polycyclique ayant une configuration plane et possédant des doubles liaisons photoactivables, ou un groupe azido-N₃.

5. Procédé selon la revendication 4, caractérisé en ce que le radical Z est choisi parmi les dérivés du 8-méthoxypsoralène, du 5-méthoxypsoralène, du 4'hydroxyméthyl-4,5',8-triméthylpsoralène, du 3-carbethoxypsoralène, de l'angelicine, du pyrido(3,4-c)psoralène, du pyrido (3,4-c)8-méthylpsoralène.

6. Procédé selon la revendication 4, caractérisé en ce que le radical Z est un groupe oxy-8-psoralène ou un groupe oxy-5-psoralène.

7. Procédé selon l'une des revendications 3 à 6, caractérisé en ce que n est un nombre entier compris entre 10 et 30.

8. Procédé selon l'une des revendications 3 à 7, caractérisé en ce que dans la formule (I) le radical B situé à l'extrémité (5') de l'oligonucléotide amorce est une adénine.

9. Procédé selon l'une des revendications 3 à 7, caractérisé en ce que dans la formule (I) au moins deux des radicaux B successifs situés à l'extrémité (5') de l'oligonucléotide amorce sont des adénines.

10. Procédé selon l'une des revendications 2 à 9, caractérisé en ce que l'un seulement des oligonucléotides amorces est conjugué à un ou plusieurs agents intercalants photoactivables.

11. Procédé de détection de mutations par électrophorèse d'ADN double brin en gradient de dénaturation, selon l'une des revendications 2 à 10, caractérisé en ce que l'on effectue une amplification génétique, dont les phases d'hybridation sont effectuées avec deux oligonucléotides amorces sur l'un desquels au moins se trouve fixé un ou plusieurs groupes intercalants photoactivables, du fragment d'ADN à analyser, puis en ce que l'on soumet les hybrides obtenus à une irradiation, et en ce que l'on détecte d'éventuelles mutations en comparant le résultat de l'électrophorèse de retardement sur gel en gradient de dénaturation desdits hybrides préalablement irradiés avec l'électrophorèse dans les mêmes conditions d'un échantillon d'ADN témoin dépourvu de mutation ou comportant une mutation connue.

12. Procédé selon la revendication 11, caractérisé en ce que l'électrophorèse de retardement sur gel en gradient de dénaturation est effectuée selon un gradient de température ou un gradient de dénaturant chimique ou encore un gradient de température dans le temps.

13. Application d'un fragment d'ADN double brin stabilisé par photopontage au moyen d'au moins un agent intercalant photoactivé, pour la détection de mutations par électrophorèse de retarcement sur gel en gradient de dénaturation, dans laquelle le fragment d'ADN double brin a été préparé par un processus d'amplification génétique dont les phases d'hybridation sont effectuées avec deux oligonucléotides amorces sur l'un desquels au moins se trouve fixé un ou plusieurs agents intercalants photoactivables, ladite stabilité par photopontage du fragment d'ADN double brin étant supérieure à celle d'au moins un domaine de dénaturation portant la ou les mutations analysées éventuellement présent dans l'ADN.

## Patentansprüche

1. Verfahren zum Nachweis von Mutationen durch Elektrophorese des DNS-Doppelstrangs über Denaturierungsgradienten, dadurch gekennzeichnet, dass mindestens ein Ende des analysierten DNS-Doppelstrangfragments durch Photoverbrückung mit einem oder mehreren photoaktivierten Abstandshaltern stabilisiert ist, wobei die sogennante Stabilität durch Photoverbrückung des mindestens einen Ende dieser DNS derjenigen von den mindestens einem der in DNS eventuell vorliegenden, die analysierte Mutation oder Mutationen tragenden, denaturierten Bereiche überlegen ist.

2. Verfahren nach Anspruch 1, dadurch gekennzeichnet, daß die analysierten DNS-Doppelstrangfragmente über einen genetischen Amplifikationssprozeß aus einem DNS-Doppelstrangfragment gewonnen werden, dessen Hybridphasen mit zwei Starter-Oligonukleotiden durchgeführt werden, von denen mindestens einer an einem oder mehreren der photoaktivierbaren Abstandshalter fixiert ist.

3. Verfahren nach Anspruch 2, dadurch gekennzeichnet, daß die Gruppe, bestehend aus einem Starter-Oligonukleotiden, an dem ein oder mehrere photoaktivierbare Abstandshalter der nachstehenden Formel entspricht : 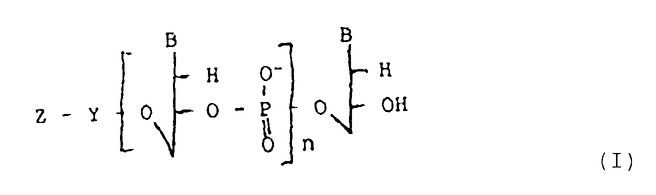 wobei in dieser :
Die Reste B identisch oder verschieden sein können und unabhängig voneinander eine Base einer Nukleinsäure darstellen.
Y einen linearen oder verzweigten Alkylrest -alk- oder einen Rest : 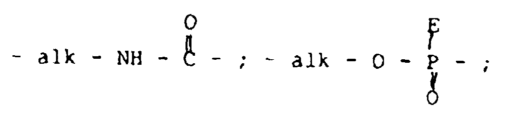 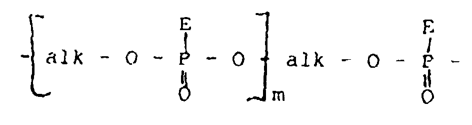
Die Reste E können identisch oder verschieden sein und stellen unabhängig voneinander ein Oxoanion O⁻, ein Thioanion S⁻, eine Alkoxygruppe, oder eine Gruppe -O-alk-Z dar; und m ist eine ganze Zahl zwischen 1 und 5.
Z stellt einen Rest dar, der einem fotoaktivierbaren Abstandshalter entspricht, welcher Photoverbrückungen mit pyrimidischen Basen bilden kann.
n ist eine ganze Zahl zwischen 2 und 150.

4. Verfahren nach Anspruch 3, dadurch gekennzeichnet, daß der Rest Z eine polyzyklische Verbindung ist, mit einer planaren Konfiguration und die fotoaktivierbare Doppelbindungen aufweist, oder eine Azido-N₃-Gruppe.

5. Verfahren nach Anspruch 4, dadurch gekennzeichnet, daß der Rest Z ausgewählt wird aus Derivaten von 8-Methoxypsoralen, 5-Methoxypsoralen, 4'-hydroxymethyl-4,5',8-triméthylpsoralen, oder 3-Carbethoxy-psolaren, Angelizin, Pyrido(3,4-c)psolaren, Pyrido(3,4-c)8-methylpsolaren.

6. Verfahren nach Anspruch 4, dadurch gekennzeichnet, daß der Rest Z eine Oxy-8-psoralen- oder Oxy-5-psolarengruppe ist.

7. Verfahren nach einem der Ansprüche 3 bis 6, dadurch gekennzeichnet, daß n eine ganze Zahl zwischen 10 und 30 ist.

8. Verfahren nach einem der Ansprüche 3 bis 7, dadurch gekennzeichnet, daß in Formel (I) der Rest B, am 5'-Ende des Oligonukleotids angeordnet, ein Adenin ist.

9. Verfahren nach einem der Ansprüche 3 bis 7, dadurch gekennzeichnet, daß in der Fromel (I) mindestens zwei der aufeinanderfolgenden Reste B, am 5'-Ende des Oligonukleotids angeordnet, Adenine sind.

10. Verfahren nach einem der Ansprüche 2 bis 9, dadurch gekennzeichnet, daß nur einer der Starter-Oligonukleotide an einem oder mehreren fotoaktivierbaren Abstandshalter konjugiert ist.

11. Verfahren zum Nachweis von Mutationen durch Elektrophorese über Denaturierungsgradienten an doppelsträngiger DNS, nach einer der Ansprüche 2 bis 10, dadurch gekennzeichnet, daß eine Genamplifikation durchgeführt wird, deren Hybridisierungsphasen mit zwei Starter-Oligonukleotiden durchgeführt werden, wobei an mindestens einem dieser ein oder mehrere fotoaktivierbare Abstandshalter fixiert sind, des zu analysierenden DNS-Fragments, und daß dann die erhaltenen Hybride einer Strahlung ausgesetzt werden und daß gegebenfalls vorhandene Mutationen nachgewiesen werden, indem die Ergebnisse der verzögerten Elektrophorese auf Gel, über Denaturierungsgradienten der beschriebenen, vorher bestrahlten Hybride mit der Elektrophorese einer DNS-Vergleichsprobe ohne Mutation oder mit einer bekannten Mutation unter gleichen Bedingungen verglichen werden.

12. Verfahren nach Anspruch 11, dadurch gekennzeichnet, daß die verzögerte Elektrophorese auf Gel über Denaturierungsgradienten nach einem Temperaturgradienten, einem Gradienten aus chemischen Denaturierer oder auch einem Temperaturgradienten mit der Zeit durchgeführt wird.

13. Verwendung eines über Fotoverbrückung mit mindestens einem fotoaktivierten Abstandshalter stabilisierten DNS-Doppelstrangfragments zum Nachweis von Mutationen durch verzögerte Gelelektrophorese über Denaturierungsgradienten, wobei das DNS-Doppelstrangfragment durch einen Genamplifakationsprozeß hergestellt wird, dessen Hybridisierungsphasen mit zwei Starter-Oligonukleotiden durchgeführt werden, wobei an mindestens einem dieser eine oder mehrere fotoaktivierbare Abstandshalter fixiert sind, wobei die sogenannte Stabilität durch Photoverbrückung des mindestens einen Ende dieser DNS derjenigen von den mindestens einem der in DNS eventuell vorliegenden, die analysierte Mutation oder Mutationen tragenden, denaturierten Bereiche überlegen ist.

## Claims

1. Process for detecting mutations by electrophoresis of double-stranded DNA in denaturation gradient, characterized in that at least one of the extremities of the analyzed fragments of double-stranded DNA is stabilized by photo-coupling using one or more photo-activated interconnecting agents, with said stability through photo-coupling of at least one of the said DNA extremities being greater than that of at least one denaturation area carrying the analyzed mutation or mutations which may be present in the DNA.

2. Process according to Claim 1, characterized in that the extremities of the analyzed fragments of double-stranded DNA are obtained by a process of genetic amplification of a fragment of double-stranded DNA, whose hybridization phases are carried out with two starter oligonucleotides on at least one of which one or more photo-activable interconnecting agents are fixed.

3. Process according to Claim 2, characterized in that the assembly made up of a starter oligonucleotide to which one or more photo-activable interconnecting agents are fixed corresponds to the following formula: 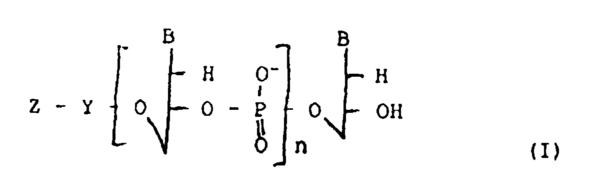 in which:
Radicals B may be identical or different and each represent a nucleic acid base.
Y represents a straight or branched alkylene radical - alk - or a radical: 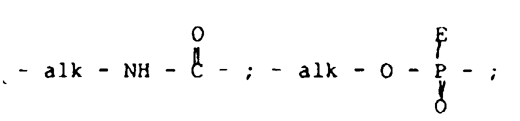 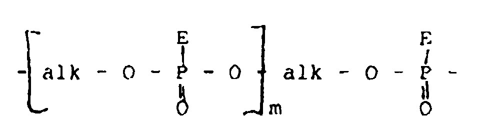
Radicals E may be identical or different and each represent an oxoanion 0⁻, a thioanion S⁻, an alkoxy group, or a group - O - alk - Z; and m is a whole number between 1 and 5.
Z is a radical corresponding to a photo-activable interconnecting agent capable of forming photo-couplings with the pyrimidine bases.
n is a whole number between 2 and 150.

4. Process according to Claim 3, characterized in that radical Z is a polycyclic compound having a flat configuration and possessing double photo-activable bonds, or an azido-N₃ group.

5. Process according to Claim 4, characterized in that radical Z is chosen from among the derivatives of 8-methoxypsoralene, 5-methoxypsoralene, 4' hydroxymethyl-4,5', 8-trimethylpsoralene, 3-carbethoxypsoralene, angelicine, pyrido (3,4-c)psoralene, pyrido (3,4-c) 8-methylpsoralene.

6. Process according to Claim 4, characterized in that radical Z is an oxy-8-psoralene group or an oxy-5-psoralene group.

7. Process according to one of Claims 3 to 6, characterized in that n is a whole number between 10 and 30.

8. Process according to one of Claims 3 to 7, characterized in that, in formula (I), radical B situated at the extremity (5') of the starter oligonucleotide is an adenine.

9. Process according to one of Claims 3 to 7, characterized in that, in formula (I), at least two of the successive radicals B at the extremity (5') of the starter oligonucleotide are adenines.

10. Process according to one of Claims 2 to 9, characterized in that one only of the starter oligonucleotides is conjugated with one or more photo-activable interconnecting agents.

11. Process for detecting mutations by electrophoresis of double-stranded DNA in denaturation gradient, according to one of Claims 2 to 10, characterized in that a genetic amplification is performed, whose hybridization phases are carried out with two starter oligonucleotides on at least one of which one or more photo-activable interconnecting agents are fixed, of the DNA fragment to be analyzed, and then in that the hybrids obtained are submitted to radiation, and in that any mutations are detected by comparing the delayed gel electrophoresis in denaturation gradient of the said previously radiated hybrids with the electrophoresis in the same conditions of a sample of control DNA devoid of mutation or including a known mutation.

12. Process according to Claim 11, characterized in that the delayed gel electrophoresis in denaturation gradient is carried out according to a temperature gradient or a chemical denaturant gradient or again a temperature gradient in time.

13. Application of a fragment of double-stranded DNA stabilized by photo-coupling through at least one photo-activated interconnecting agent, for the detection of mutations by delayed gel electrophoresis in denaturation gradient, in which the fragment of double-stranded DNA has been prepared by a process of genetic amplification whose hybridization phases are carried out with two starter oligonucleotides, on at least one of which one or more photo-activable interconnecting agents are fixed, with said stability through photo-coupling of the fragment of double-stranded DNA being greater than that of at least one denaturation area carrying the analyzed mutation or mutations which may be present in the DNA.
